(19) Europäisches Patentamt
European Patent Office
Office européen des brevets

(11) **EP 2 829 232 A1**

(12) **EUROPÄISCHE PATENTANMELDUNG**

(43) Veröffentlichungstag:
28.01.2015 Patentblatt 2015/05

(51) Int Cl.:
*A61B 6/00* (2006.01)      *G01N 23/04* (2006.01)

(21) Anmeldenummer: 14178229.2

(22) Anmeldetag: **23.07.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **24.07.2013   DE 102013214482**

(71) Anmelder: **Fraunhofer-Gesellschaft zur Förderung der angewandten Forschung e.V.**
**80686 München (DE)**

(72) Erfinder:
• **Sukowski, Frank**
  **92353 Postbauer-Heng (DE)**
• **Schröpfer, Stefan**
  **90522 Oberasbach (DE)**

• **Schön, Tobias**
  **90453 Nürnberg (DE)**
• **Fuchs, Theobald**
  **90429 Nürnberg (DE)**
• **Hassler, Ulf**
  **91560 Heilsbronn (DE)**
• **Tigkos, Konstantinos**
  **90425 Nürnberg (DE)**
• **Schorr, Christian**
  **66636 Tholey (DE)**
• **Maisl, Michael**
  **66629 Freisen (DE)**

(74) Vertreter: **Hersina, Günter**
**Schoppe, Zimmermann, Stöckeler, Zinkler & Partner**
**Postfach 246**
**82043 Pullach (DE)**

(54) **Prüfkörper für ein 3D-Röntgensystem und Verfahren zur Bestimmung der Ortsauflösung eines 3D-Röntgensystems mittels des Prüfkörpers**

(57)   Ein Prüfkörper 10 zur Bestimmung der Ortsauflösung eines 3D-Röntgensystems, weist drei orthogonal zueinander angeordnete und miteinander verbundene Scheibenstrukturen 12, 14, 16 mit einem gemeinsamen Mittelpunkt 18 auf, wobei jede Scheibenstruktur eine geradzahlige Anzahl N von Pyramiden- oder Pyramidenstumpf-förmigen Scheibenstruktursektoren 12-N, 14-N, 16-N aufweist, die sich zu dem gemeinsamen Mittelpunkt hin verjüngen und wobei jeweils benachbarte Scheibenstruktursektoren einer Scheibenstruktur unterschiedliche Röntgenstrahlungsabsorptionseigenschaften aufweisen.

FIG 1A

**Beschreibung**

Die vorliegende Erfindung bezieht sich auf einen Prüfkörper zur Bestimmung der Ortsauflösung eines 3D-Röntgensystems, auf ein Verfahren zur Bestimmung der Ortsauflösung eines 3D-Röntgensystems mittels des Prüfkörpers und auf die Verwendung des Prüfkörpers zur Bestimmung der Ortsauflösung eines 3D-Röntgensystems. Insbesondere bezieht sich die vorliegende Erfindung auf einen Prüfkörper zur Evaluierung des Ortsauflösungsvermögens eines 3D-Röntgenscanners in zumindest einer von drei orthogonalen Raumrichtungen. Der Prüfkörper wird hierbei von einer Röntgenanlage mittels eines 3D-Scanverfahrens, wie z.B. mittels Computertomographie (CT) oder Computerlaminographie (CL), aufgenommen, woraufhin der 3D-Volumendatensatz rekonstruiert wird. Anhand dieses Datensatzes lässt sich das Ortsauflösungsvermögen des resultierenden 3D-Scans, d.h. der resultierenden 3D-Röntgenaufnahme, in drei orthogonal zueinander stehenden Raumebenen bestimmen.

Derzeit bekannte Prüfkörper zur simultanen Bestimmung der Ortauflösung einer 3D-Röntgenanlage in drei Raumrichtungen basieren alle auf sogenannten Balkengruppentests oder Lochmustern in einer Ebene, wobei in jeder Raumrichtung die Ortsauflösung durch eine gesonderte Messung nach vorheriger Ausrichtung des Prüfkörpers bestimmt werden muss. Die Balkengruppentestmuster sind insbesondere teuer in der Anschaffung und nur mit einem sehr hohen Aufwand korrekt in der Röntgenanordnung positionierbar, da sie vor der Messung und ggf. innerhalb eines Behältnisses, z.B. eines Luftfrachtcontainers etc., exakt in der Ebene ausgerichtet sein müssen, in der aus dem Volumendatensatz anschließend die Bildqualität quantifiziert werden soll.

Die der vorliegenden Erfindung zugrunde liegende Aufgabe besteht nun darin, einen verbesserten Prüfkörper zur Bestimmung der Ortsauflösung in bis zu drei orthogonalen Raumrichtungen eines 3D-Röntgensystems und ein entsprechendes Verfahren zur Bestimmung der Ortsauflösung zu schaffen.

Diese Aufgabe wird durch einen Prüfkörper zur Bestimmung der Ortsauflösung eines 3D-Röntgensystems gemäß Anspruch 1, durch ein Verfahren zur Bestimmung der Ortsauflösung eines 3D-Röntgensystems gemäß Anspruch 9 und durch die Verwendung des Prüfkörpers zur Bestimmung der Ortsauflösung eines 3D-Röntgensystems in zumindest einer von drei orthogonalen Raumrichtungen gemäß Anspruch 10 gelöst.

Erfindungsgemäße Weiterbildungen sind in den Unteransprüchen definiert.

Der Kerngedanke der vorliegenden Erfindung besteht nun darin, einen dreidimensionalen Prüfkörper aus drei aufeinander orthogonal stehenden Scheiben bzw. Scheibenstrukturen zusammenzusetzen, wobei die Scheiben bzw. Scheibenstrukturen wiederum aus Pyramiden-förmigen bzw. Pyramidenstumpf-förmigen Elementen zusammengesetzt sind. Aufgrund der sich verjüngenden (spitz zulaufenden) Pyramiden verjüngen sich die Scheiben zur Mitte bzw. Mittelachse hin. Die Pyramiden-förmigen Elemente sind nun so angeordnet, dass sich entlang des Umfangs einer jeden Scheibe die Materialien regelmäßig abwechseln.

Im Fall von zwei Materialien bedeutet dies, dass sich diese beiden unterschiedlichen Werkstoffe jeweils abwechseln. Der dreidimensionale Prüfkörper kann also eine Aneinanderreihung mehrerer Kreis- oder Kreisringsektoren aufweisen, die abwechselnd aus verschiedenen Materialien gebildet sein können.

Ferner können benachbarte Scheibenstruktursektoren einer Scheibenstruktur abwechselnd einen physischen Scheibenstruktursektor und einen als Materialaussparung ausgebildeten Scheibenstruktursektor aufweisen. Der dreidimensionale Prüfkörper kann also eine Aneinanderreihung mehrerer Kreis- oder Kreisringsektoren aufweisen, die abwechselnd aus Scheibenstruktursektoren und Scheibenstruktursektor-förmigen Freiräumen gebildet sein können.

Ganz allgemein kann eine beliebige Anzahl von Materialien N aneinandergereiht werden, wobei beispielsweise zwei verschiedene Werkstoffe (d.h. Material 1, Material 2) in benachbarten Scheibensektoren eingesetzt werden. Um nun einen möglichst starken Kontrast im resultierenden Röntgenbild zwischen den verschiedenen Materialien der Sektoren und damit zwischen den einzelnen Sektoren zu erzielen, weist im Fall von $N = 2$ die eine Hälfte der Sektoren das Material 1 auf, d.h. üblicherweise ein schweres Material, wie z.B. Stahl, Buntmetall, Schwermetall etc., mit einem hohen Absorptionskoeffizienten (auch Dämpfungskonstante oder linearer Schwächungskoeffizient) für Röntgenstrahlung, während die anderen Sektoren das Material 2, d.h. ein leichtes Material wie z.B. Kunststoff, Leichtmetall etc., mit einem niedrigen Absorptionskoeffizienten für Röntgenstrahlung aufweisen.

Alternativ können benachbarte Scheibenstruktursektoren einer Scheibenstruktur abwechselnd einen physischen Scheibenstruktursektor (Material 1) und eine Scheibenstruktursektor-förmige Materialaussparung (bzw. einen Materialfreiraum- oder Zwischenraum) aufweisen. Somit kann zwischen zwei Pyramiden-förmig bzw. Pyramidenstumpf-förmig ausgebildeten, physischen Elementen (Scheibenstruktursektoren) eine Scheibenstruktursektor-förmige Aussparung oder Ausnehmung (bzw. ein Freiraum) vorgesehen sein, um die entlang des Umfangs einer Scheibenstruktur regelmäßig abwechselnde Anordnung ("physischer Scheibenstruktursektor" - "Freiraum" - "physischer Scheibenstruktursektor" - "Freiraum" - etc.) zu erhalten.

Die physischen Scheibenstruktursegmente können beispielsweise einen Werkstoff (z.B. ein Material 1) oder untereinander auch unterschiedliche Werkstoffe aufweisen. Das Material bzw. die Materialien für die Pyramiden-förmigen bzw. Pyramidenstumpf-förmigen Elemente sind nun so gewählt, um wiederum einen möglichst starken Kontrast im resultierenden Röntgenbild zwischen den physischen Scheibenstruktursektoren und den jeweils dazwischenliegenden Pyramiden-förmigen bzw. Pyramidenstumpf-förmigen Aussparungen (bzw. Freiräumen, Leerstellen oder Lücken) zu erzielen. D.h., das Material bzw. die Materialien für die physischen Scheibenstruktursektoren weisen einen (deutlich) höheren Absorptionskoeffizienten (auch Dämpfungskonstante oder linearer Schwächungskoeffizient) für Röntgenstrahlung als die Freiräume (z.B. Luft oder Umgebungsatmosphäre) auf.

Der dreidimensionale Prüfkörper weist drei aufeinander orthogonal stehende Scheiben bzw. Scheibenstrukturen auf, wobei die einzelnen Scheibenstrukturen einen im Wesentlichen gleichen Aufbau aufweisen können. Alternativ können die Scheibenstrukturen eines Prüfkörpers hinsichtlich der gewählten Materialien und des Aufbaus auch unterschiedlich ausgebildet sein. So können benachbarte Scheibenstruktursektoren bei einer Scheibenstruktur unterschiedliche Materialien aufweisen, während bei einer der weiteren Scheibenstrukturen die benachbarten Scheibenstruktursektoren abwechselnd einen physischen Scheibenstruktursektor und einen als Materialaussparung ausgebildeten Scheibenstruktursektor aufweisen können.

Ausführungsbeispiele beschreiben somit einen Prüfkörper zur Bestimmung der Ortsauflösung eines 3D-Röntgensystems, wobei der Prüfkörper drei orthogonal zueinander angeordnete und miteinander verbundene Scheibenstrukturen mit einem gemeinsamen Mittelpunkt aufweist. Jede Scheibenstruktur weist eine geradzahlige Anzahl N von Pyramiden- oder Pyramidenstumpf-förmigen Scheibenstruktursektoren auf, die sich zu dem gemeinsamen Mittelpunkt hin verjüngen, wobei jeweils benachbarte Scheibenstruktursektoren unterschiedliche Röntgenstrahlungsabsorptionseigenschaften aufweisen. Dabei können benachbarte Scheibenstruktursektoren einer Scheibenstruktur unterschiedliche Materialien aufweisen. Ferner (alternativ oder zusätzlich) können benachbarte Scheibenstruktursektoren einer Scheibenstruktur abwechselnd einen physischen Scheibenstruktursektor und einen als Materialaussparung (bzw. Materialfreiraum) ausgebildeten Scheibenstruktursektor aufweisen.

Ausführungsbeispiele beschreiben somit ferner ein Verfahren zur Bestimmung der Ortsauflösung eines 3D-Röntgensystems mittels des Prüfkörpers, mit folgenden Schritten: Platzieren des Prüfkörpers in einer Prüfposition zwischen einer Röntgenquelle und einem Röntgendetektor des 3D-Röntgensystems; Erfassen einer Abfolge von Durchstrahlungsaufnahmen des Prüfkörpers an unterschiedlichen Positionen (z.B. Winkelpositionen oder Längspositionen), um eine Gesamtzahl von Mess-Durchstrahlungsaufnahmen zu erhalten; Rekonstruieren einer 3D-Röntgenaufnahme des Prüfkörpers basierend auf der Gesamtzahl von Mess-Durchstrahlungsaufnahmen; und Bestimmen der Ortsauflösung des 3D-Röntgensystems basierend auf dem Durchmesser d eines Unschärfe-Bereichs der 3D-Röntgenaufnahme im Zentrum des Prüfkörpers, wobei innerhalb des Unschärfe-Bereichs keine Auflösung der einzelnen Scheibenstruktursektoren vorliegt, und auf der Anzahl N von Pyramiden- oder Pyramidenstumpf-förmigen Scheibenstruktursektoren.

Die Ortsauflösung "l" des 3D-Röntgensystems wird beispielsweise basierend auf der folgenden Beziehung ermittelt:

$$l = \pi \cdot d/N;$$

mit dem Durchmesser d des Unschärfe-Bereichs der 3D-Röntgenaufnahme im Zentrum des Prüfkörpers und der Anzahl N von Scheibenstruktursektoren pro Scheibenstruktur.

Ausführungsbeispiele beschreiben ferner die Verwendung des Prüfkörpers zur Bestimmung der Ortsauflösung eines 3D-Röntgensystems in zumindest einer von drei orthogonalen Raumrichtungen.

Bevorzugte Ausführungsbeispiele der vorliegenden Erfindung werden nachfolgend Bezug nehmend auf die beiliegenden Zeichnungen näher erläutert. Es zeigen:

Fig. 1a     eine Prinzipdarstellung des Prüfkörpers zur Bestimmung der Ortsauflösung eines 3D-Röntgensystems gemäß einem Ausführungsbeispiel der vorliegenden Erfindung;

Fig. 1b     eine Prinzipdarstellung in mehreren Ansichten eines einzelnen Scheibensektors des Prüfkörpers mit einer beispielhaften Bemaßung gemäß einem Ausführungsbeispiel der vorliegenden Erfindung;

Fig. 2     ein Flussdiagramm eines Verfahrens zur Bestimmung der Ortsauflösung eines 3D-Röntgensystems mittels des Prüfkörpers in zumindest einer von drei orthogonalen Raumrichtungen gemäß einem Ausführungsbeispiel der vorliegenden Erfindung; und

Fig. 3a     eine Prinzipdarstellung eines 3D-Röntgensystems zur Durchführung des erfindungsgemäßen Verfahrens zur Bestimmung der Ortsauflösung des 3D-Röntgensystems gemäß einem Ausführungsbeispiel der vorliegenden Erfindung; und

Fig. 3b     beispielhafte 3D-Röntgenaufnahmen mit unterschiedlich resultierenden Ortsauflösungen hinsichtlich einer Raumrichtung des 3D-Röntgensystems.

Bevor nachfolgend Ausführungsbeispiele der vorliegenden Erfindung im Detail anhand der Zeichnungen näher erläutert werden, wird darauf hingewiesen, dass identische, funktionsgleiche oder gleichwirkende Elemente, Objekte und/oder Strukturen in den unterschiedlichen Figuren mit den gleichen Bezugszeichen versehen sind, so dass die in den unterschiedlichen Ausführungsbeispielen dargestellte Beschreibung dieser Elemente untereinander austauschbar ist bzw. aufeinander angewendet werden kann.

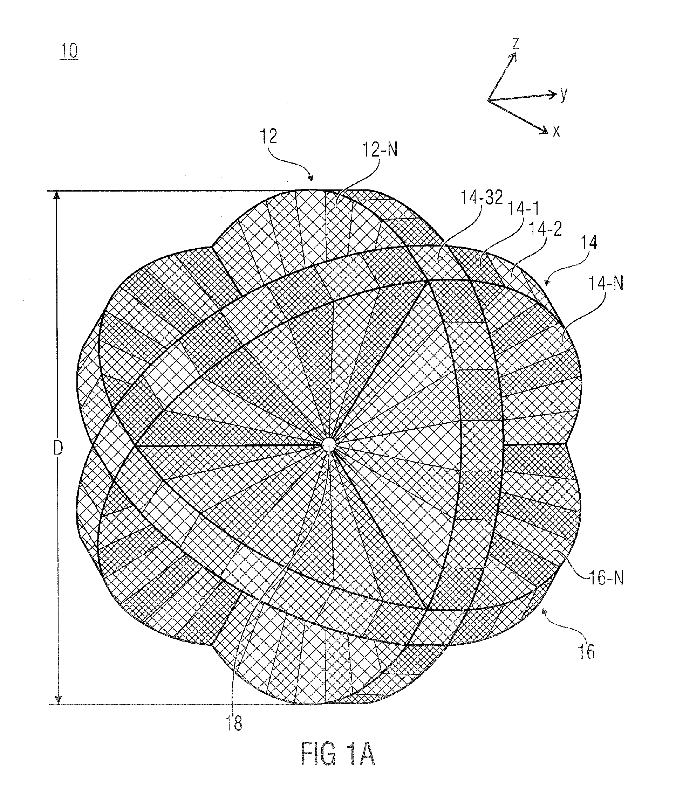

Im Folgenden wird nun anhand der in Fig. 1a gezeigten Prinzipdarstellung ein Prüfkörper 10 zur Bestimmung der Ortsauflösung eines 3D-Röntgensystems (nicht gezeigt in Fig. 1a) beschrieben.

Der Prüfkörper 10 zur Bestimmung der Ortsauflösung in zumindest einer von drei orthogonalen Raumrichtungen bzw. in bis zu drei orthogonalen Raumrichtungen eines 3DRöntgensystems umfasst drei orthogonal zueinander angeordnete und miteinander verbundene Scheibenstrukturen 12, 14, 16 mit einem gemeinsamen Mittelpunkt 18. Jede Scheibenstruktur 12, 14, 16 weist eine geradzahlige Anzahl N von Pyramiden- oder Pyramidenstumpf-förmigen Scheibenstruktursektoren 12-N, 14-N, 16-N auf, die sich zu dem gemeinsamen Mittelpunkt 18 hin verjüngen. Die einzelnen Scheibenstruktursektoren 12-N, 14-N, 16-N können somit als Kreissektoren (bzw. Kreisringsektoren) oder Sektoren eines regelmäßigen N-Ecks angesehen werden. Als regelmäßiges oder reguläres N-Eck wird im Rahmen der vorliegenden Beschreibung ein N-Eck mit gleichen Seiten und gleichen Innenwinkeln angesehen, das ferner isogonal ist, d.h. seine Ecken liegen gleichabständig, also unter gleichem Zentriwinkel auf einem Kreis.

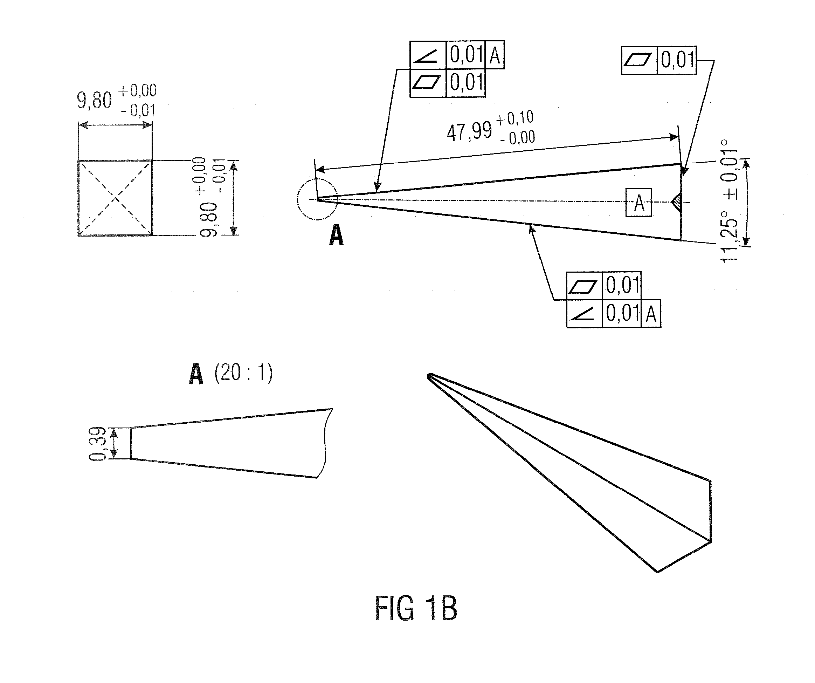

Wie aus Fig. 1a ferner ersichtlich ist, weisen jeweils zwei der drei orthogonal zueinander angeordnete und miteinander verbundene Scheibenstrukturen 12, 14 und 16 in den jeweiligen orthogonalen Raumrichtungen jeweils einen Pyramiden- oder Pyramidenstumpf-förmigen Scheibenstruktursektor gemeinsam auf. Wie in Fig. 1 a dargestellt ist, weist jede Scheibenstruktur 12, 14, 16 beispielsweise 32 zugehörige Scheibenstruktursektoren 12-N, 14-N, 16-N (d.h. hier mit N = 32) auf, wobei jeweils sechs Scheibenstruktursektoren von zwei sich orthogonal schneidenden Scheibenstrukturen an den Schnitt- bzw. Kontaktbereichen gemeinsam verwendet werden. Der Prüfkörper 10 kann somit als symmetrisch hinsichtlich drei orthogonalen Raumrichtungen angesehen werden.

Jeweils benachbarte Scheibenstruktursektoren einer Scheibenstruktur weisen unterschiedliche Röntgenstrahlungsabsorptionseigenschaften auf, um einen Kontrast im resultierenden Röntgenbild zwischen den benachbarten Scheibenstruktursektoren zu erhalten.

Somit können beispielsweise benachbarte Scheibenstruktursektoren einer Scheibenstruktur unterschiedliche Materialien oder Materialeigenschaften aufweisen, die zu unterschiedlichen Röntgenstrahlungsabsorptionseigenschaften der benachbarten Scheibenstruktursektoren führen, um einen möglichst starken Kontrast im resultierenden Röntgenbild zwischen den benachbarten Scheibenstruktursektoren zu erhalten.

Um nun einen möglichst starken Kontrast im resultierenden Röntgenbild zwischen den verschiedenen, benachbarten Sektoren zu erzielen, wird abwechselnd ein Material mit einem hohen Absorptionskoeffizienten für Röntgenstrahlung und ein weiteres Material mit einem niedrigen Absorptionskoeffizienten für Röntgenstrahlung für benachbarte Sektoren eingesetzt.

Der dreidimensionale Prüfkörper weist also eine Aneinanderreihung mehrerer Kreissektoren auf, die abwechselnd aus verschiedenen Materialien gebildet sein können. Ganz allgemein kann eine beliebige Anzahl von Materialien N aneinandergereiht werden, wobei beispielsweise im einfachsten Fall zwei verschiedene Werkstoffe (d.h. Material 1, Material 2) in benachbarten Scheibensektoren eingesetzt werden. Um nun einen möglichst starken Kontrast im resultierenden Röntgenbild zwischen den verschiedenen Materialien der Sektoren und damit zwischen den einzelnen Sektoren zu erzielen, weist im Fall von N = 2 die eine Hälfte der Sektoren das Material 1 auf, d.h. üblicherweise aus einem schweren Material, wie z.B. Stahl, Buntmetall, Schwermetall etc., mit einem hohen Absorptionskoeffizienten für Röntgenstrahlung, während die anderen Sektoren das Material 2, d.h. ein leichtes Material wie z.B. Kunststoff, Leichtmetall etc., mit einem niedrigen Absorptionskoeffizienten für Röntgenstrahlung aufweisen. Als Kunststoffmaterial für einen Scheibenstruktursektor (für ein leichtes Material 2) kann beispielsweise ein Polyaryletherketon-Kunststoff angegeben werden.

Jede Scheibenstruktur 12, 14, 16 weist somit zumindest zwei unterschiedliche und bis zu N-unterschiedliche Materialien mit Röntgenstrahlabsorptionseigenschaften auf.

Wie dies in Fig. 1a dargestellt, sind die nebeneinander angeordneten (bzw. aneinander angrenzende) und miteinander mechanisch verbundenen Scheibenstruktursektoren geometrisch gleich (bzw. identisch) ausgebildet, d.h. sie weisen gleiche geometrische Abmessungen auf. So können beispielsweise die Pyramiden- oder Pyramidenstumpf-förmigen Scheibenstruktursektoren in einer Ebene senkrecht zum Radius (ausgehend von dem gemeinsamen Mittelpunkt 18) der jeweiligen Scheibenstruktur 12, 14, 16 eine rechteckige oder quadratische Querschnittsfläche aufweisen. Somit wird beispielsweise auch die Grundfläche und bei einem Pyramidenstumpf-förmigen Scheibenstruktursektor auch die Deckfläche, die senkrecht zu Höhe der Scheibenstruktursektoren verlaufen, beispielsweise eine quadratische oder rechteckige Form aufweisen. Die außenseitige Grundfläche kann beispielsweise flach und in einer Ebene senkrecht zum Radius der jeweiligen Scheibenstruktur ausgebildet sein. Somit werden die einzelnen Sektoren der Scheibenstruktur ein regelmäßiges N-Eck ergeben.

Es ist gleichermaßen möglich, dass die außenseitigen Grundflächen der Sektoren jeweils abgerundet sind und sich eine beispielsweise kreisförmige Außenform der jeweiligen Scheibenstruktur ergibt, so dass die einzelnen Pyramiden- oder Pyramidenstumpf-förmigen Sektoren der Scheibenstrukturen als Kreis- oder Kreisringsektoren ausgebildet sind. Die Scheibenstrukturen weisen ferner ein Durchmesser D zwischen gegenüberliegenden Grundflächen der Pyramiden- oder Pyramidenstumpf-förmigen Sektoren auf.

Bezüglich der in Fig. 1a dargestellten geometrischen Ausgestaltung des Prüfkörpers 10 wird darauf hingewiesen, dass generell keine Beschränkungen bezüglich Anzahl und Höhe der Scheibenstruktursektoren vorliegen. Vielmehr kann der erfindungsgemäße Prüfkörper an die gesamte Spannbreite der industriellen oder medizinischen Computertomographiesysteme bzw. Computerlaminographiesysteme adaptiert werden. Je nach erzielbarer Ortsauflösung können die Pyramidendurchmesser, d.h. die Durchmesser der Pyramiden- oder Pyramidenstumpf-förmigen Scheibenstruktursektoren, am gemeinsamen Mittelpunkt der Scheibenstrukturen an der Spitze im Zehntel-Millimeterbereich z.B. für μCT-Anlagen bis im Zentimeterbereich für XXL-CT-Anlagen liegen, wenn vorausgesetzt wird, dass sich die "Sternstruktur" des Prüfkörpers 10 mit einer ausreichenden Genauigkeit fertigen lässt.

Somit kann angegeben werden, dass die Funktionalität des erfindungsgemäßen Prüfkörpers 10 im Wesentlichen rein auf der geometrischen Form des Prüfkörpers 10 basiert, unabhängig vom jeweiligen Durchmesser des Sterns, d.h. der Scheibenstrukturen (abhängig z.B. von der Höhe der Pyramiden- oder Pyramidenstumpf-förmigen Sektoren) und der Anzahl der Sektoren. Die Scheibenstruktursektoren können Pyramiden-förmig oder auch Pyramidenstumpf-förmig ausgebildet sein, wobei im Falle einer Pyramidenstumpf-förmigen Ausbildung in der Mitte der Scheibenstrukturen (an dem gemeinsamen Mittelpunkt 18) dann ein kleiner Bereich freigelassen wird.

Beispielsweise können für jede Scheibenstruktur 12, 14, 16 zumindest 16 Scheibenstruktursektoren vorgesehen sein.

Wie bereits oben angegeben wurde, können die Scheibenstruktursektoren 12-N, 14-N, 16-N Pyramidenstumpf-förmig ausgebildet sein, wobei eine Länge "a" ausgehend von der Grundfläche bis zu der Deckfläche des Pyramidenstumpf-förmigen Scheibenstruktursektors vorgesehen ist. Dabei weist die Länge "a" zumindest 80% (oder 90%) des Abstandes zwischen dem Mittelpunkt oder Flächenschwerpunkt der jeweiligen Grundfläche der Py-ramidenstumpf-förmigen Scheibenstruktursektoren bis zu dem gemeinsamen Mittelpunkt auf.

Alternativ können benachbarte Scheibenstruktursektoren einer Scheibenstruktur abwechselnd einen physischen Scheibenstruktursektor und einen Scheibenstruktursektor-förmigen Freiraum aufweisen. Somit kann zwischen zwei physischen Scheibenstruktursektoren ein Scheibenstruktursektor-förmiger Freiraum vorgesehen sein, um die entlang des Umfangs einer Scheibenstruktur regelmäßig abwechselnde Anordnung ("physischer Scheibenstruktursektor" - "Freiraum" - "physischer Scheibenstruktursektor" - "Freiraum" - etc.) zu erhalten.

Die physischen Scheibenstruktursegmente können beispielsweise einen gemeinsamen Werkstoff oder untereinander auch unterschiedliche Werkstoffe aufweisen. Das Material bzw. die Materialien für die physischen Scheibenstruktursegmente sind nun so gewählt, um wiederum einen möglichst starken Kontrast im resultierenden Röntgenbild zwischen den physischen Scheibenstruktursektoren und den jeweils dazwischenliegenden Freiräumen zu erzielen. D.h., das Material bzw. die Materialien für die physischen Scheibenstruktursektoren weisen einen (deutlich) höheren Absorptionskoeffizienten (auch Dämpfungskonstante oder linearer Schwächungskoeffizient) für Röntgenstrahlung als die Freiräume (z.B. Luft oder Umgebungsatmosphäre) auf.

Ausführungsbeispiele beschreiben somit ferner einen Prüfkörper zur Bestimmung der Ortsauflösung eines 3D-Röntgensystems, wobei der Prüfkörper drei orthogonal zueinander angeordnete und miteinander verbundene Scheibenstrukturen mit einem gemeinsamen Mittelpunkt aufweist. Jede Scheibenstruktur weist eine geradzahlige Anzahl N von Pyramiden- oder Pyramidenstumpf-förmigen Scheibenstruktursektoren auf, die sich zu dem gemeinsamen Mittelpunkt hin verjüngen, wobei benachbarte Scheibenstruktursektoren einer Scheibenstruktur abwechselnd einen physischen Scheibenstruktursektor und einen als Materialaussparung ausgebildeten Scheibenstruktursektor aufweisen.

Die im Vorhergehenden hinsichtlich Fig. 1a dargestellte Beschreibung ist somit entsprechend auf eine Ausgestaltung anwendbar, bei der benachbarte Scheibenstruktursektoren einer Scheibenstruktur abwechselnd einen physischen Scheibenstruktursektor und einen Scheibenstruktursektor-förmigen Freiraum aufweisen. Zwischen zwei physischen (körperlichen) Scheibenstruktursektoren kann nun ein entsprechend ausgebildeter (Pyramidenförmig bzw. Pyramidenstumpf-förmig) ausgebildeter Freiraum (als Aussparung, Leerstelle oder Lücke) vorgesehen sein, um die entlang des Umfangs einer Scheibenstruktur regelmäßig abwechselnde Anordnung ("Pyramiden-förmiges Material" - "Freiraum" - "Pyramiden-förmiges Material" - "Freiraum" etc.) zu erhalten.

Um nun eine mechanische Verbindung der physischen Scheibenstruktursektoren und damit die mechanische Stabilität der resultierenden Scheibenstrukturen 12, 14, 16 untereinander und auch des zusammengefügten Prüfkörper zu erhalten, kann ein mechanisches Verbindungselement (nicht gezeigt in Fig. 1 a) der physischen Scheibenstruktursektoren beispielsweise im Bereich des gemeinsamen Mittelpunkts vorgesehen werden. Dieses mechanische Verbindungselement kann beispielsweise durch ein bereichsweises Ausgießen der Materialaussparungen im Bereich des gemeinsamen Mittepunkts 18 gebildet werden. Alternativ oder zusätzlich kann an der Grundfläche der Scheibenstruktursektoren ferner "ein Ringelement" (nicht gezeigt in Fig. 1 a) vorgesehen sein, um die Grundflächen der "physischen" Scheibenstruktursektoren der jeweiligen Scheibenstrukturen mechanisch miteinander zu verbinden und eine entsprechende mechanische Stabilität der resultierenden Scheibenstrukturen bzw. des resultierenden Prüfkörpers zu erhalten.

Die oben dargestellten mechanischen Verbindungsmöglichkeiten der unterschiedlichen Scheibenstruktursektoren sind nur als beispielhaft anzusehen, wobei eine beliebige mechanische Verbindung der einzelnen Scheibenstruktursektoren der Scheibenstrukturen gewählt werden kann, um einen mechanisch stabil zusammengesetzten Prüfkörper zu erhalten.

Fig. 1b zeigt nun in unterschiedlichen Ansichten einen einzelnen Pyramiden- oder Pyramidenstumpf-förmigen Scheibenstruktursektor einer der Scheibenstrukturen 12, 14, 16. Die Bemaßungen und Größenangaben sind als rein beispielhaft anzusehen.

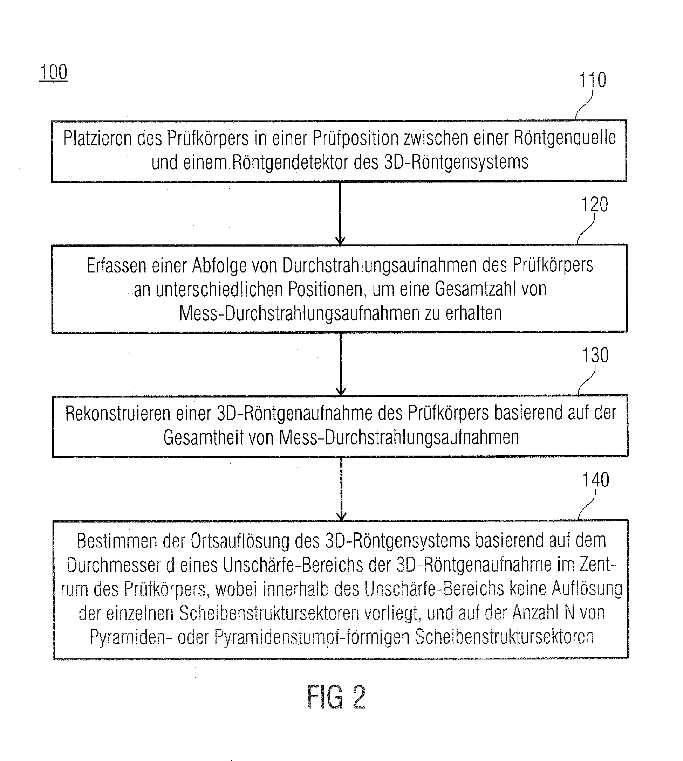

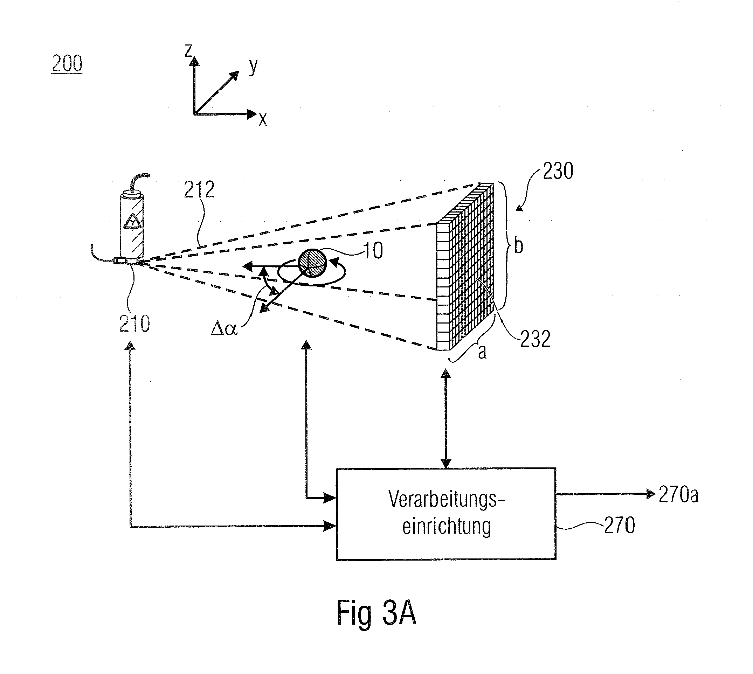

Im Folgenden wird nun anhand des in Fig. 2 dargestellten Flussdiagramms eines erfindungsgemäßen Verfahrens zur Bestimmung der Ortsauflösung eines 3D-Röntgensystems unter Verwendung des in Fig. 1a-1b dargestellten 3D-Prüfkörpers und anhand des in Fig. 3a-b dargestellten beispielhaften 3D-Röntgensystems zur Durchführung des erfindungsgemäßen Verfahrens das erfindungsgemäße Konzept zur Bestimmung der Ortsauflösung eines 3D-Röntgensystems mittels des erfindungsgemäßen 3D-Prüfkörpers näher erläutert.

Bei 3D-Röntgensystemen wird von einer Röntgenröhre als Strahlungsquelle Röntgenstrahlung in Form eines Fächerstrahls oder eines Kegelstrahls abgestrahlt, die das Untersuchungsobjekt bzw. Prüfobjekt durchdringt und auf einen Röntgendetektor fällt. Entsprechend dem Prinzip eines 3D-Röntgensystemen trifft Röntgenstrahlung auf Materie, wobei je nach Material- und Objektbeschaffenheit, z.B. Materialdichte und Durchstrahlungslängen, ein unterschiedlich großer Anteil der Strahlung von dem Untersuchungsobjekt absorbiert wird. Mittels der Durchstrahlung bzw. Projektion der Fläche des Untersuchungsobjekts unter einer Vielzahl von Winkel- oder Längspositionen lässt sich eine räumliche Information des Untersuchungsobjekts ermitteln. Je mehr Projektionen unter unterschiedlichen Winkeln oder unterschiedlichen Positionen aufgenommen werden, umso mehr Tiefeninformationen (3D-Informationen) liegen vor, die man dann aus den Aufnahmen rekonstruieren kann.

Beispielsweise stellen die Computertomographie (CT) und die Computerlaminographie (CL) häufig verwendete spezielle Aufnahmetrajektorien dar. Das vorliegende Konzept eines Prüfkörpers zur Bestimmung der Ortsauflösung eines 3D-Röntgensystems ist aber allgemein auf jegliche 3D-Scans anwendbar, d.h. auch wenn das Objekt unter mehreren völlig willkürlichen Betrachtungswinkeln durchleuchtet wird, bei denen keine Regelmäßigkeit oder ein System erkennbar ist, so lange Positionen bzw. Orientierung von Quelle und Detektor für jede einzelne Projektion bekannt sind. Die Computertomographie bildet den Spezialfall ab, bei dem sowohl die Röntgenquelle als auch der Detektor sich auf konzentrischen Kreisen oder Kreissegmenten bewegen und die Flächennormale der Detektoroberfläche immer in Richtung der Röntgenquelle zeigt. Ausnahme ist die sogenannte Helix-CT, bei sich Quelle und Detektor auf einer Schraubenbahn (Helix) um eine gemeinsame Rotationsachse bewegen.

Der Spezialfall der Computerlaminographie bildet die Aufnahmetrajektorien ab, bei denen sich Quelle und Detektor auf zwei koplanaren Ebenen bewegen, wobei sich das Objekt zwischen diesen koplanaren Ebenen befindet. Die Flächennormale des Detektors ist nicht ständig auf die Quelle ausgerichtet, sondern zeigt im Regelfall während des gesamten Scanvorgangs in eine konstante Richtung. Die Bewegung der beiden Komponenten auf diesen Ebenen ist hierbei frei wählbar (linear, kreisförmig, spiralförmig, elliptisch, rechteckig, etc.).

Bei dem erfindungsgemäßen Verfahren 100 zur Bestimmung der Ortsauflösung eines 3D-Röntgensystems mittels des Prüfkörpers 10 (siehe Fig. 1a) wird zunächst der Prüfkörper 10 in einer Prüfposition zwischen einer Röntgenquelle und einem Röntgendetektors des 3D-Röntgensystems platziert (Schritt 110). Daraufhin wird eine Abfolge von Durchstrahlungsaufnahmen des Prüfkörpers an (z.B. vorgegebenen) unterschiedlichen Winkel- oder Längspositionen erfasst (Schritt 120), um eine Gesamtzahl von Messdurchstrahlungsaufnahmen zu erhalten. Basierend auf der Gesamtzahl von Messdurchstrahlungsaufnahmen wird eine 3D-Röntgenaufnahme (d.h. beispielsweise eine Computertomographieaufnahme oder Computerlaminographieaufnahme) des Prüfkörpers basierend auf der Gesamtzahl von Messdurchstrahlungsaufnahmen rekonstruiert (Schritt 130). Schließlich wird die Ortsauflösung des 3D-Röntgensystems in zumindest einer Raumebene basierend auf dem Durchmesser d eines (etwa kreisförmigen) Unschärfe-Bereichs der 3D-Röntgenaufnahme am gemeinsamen Mittelpunkt bzw. im Zentrum des Prüfkörpers in der jeweiligen Raumebene und auf der Anzahl N von Pyramiden- oder Pyramidenstumpf-förmigen Scheibenstruktursektoren bestimmt (Schritt 140). Als Unschärfe-Bereich wird nun derjenige Bereich der 3D-Röntgenaufnahme am gemeinsamen Mittelpunkt bzw. im Zentrum des Prüfkörpers bezeichnet, innerhalb dessen keine Auflösung bzw. keine Trennung der einzelnen Scheibenstruktursektoren in der resultierenden Röntgenaufnahme möglich ist bzw. vorliegt.

Basierend auf dem ermittelten Durchmesser d der Unschärfe-Bereichs kann die Ortsauflösung "l" des 3D-Röntgensystems basierend auf der folgenden Beziehung ermittelt werden:

$$l = \pi \cdot d/N;$$

mit dem (mittleren) Durchmesser d des Unschärfe-Bereichs der 3D-Röntgenaufnahme im Zentrum des Prüfkörpers und der Anzahl N von Scheibenstruktursektoren pro Scheibenstruktur. Der Schritt des Bestimmens der Ortsauflösung kann in einer Raumebene oder in mehreren orthogonalen Raumebenen der 3d-Röntgenaufnahme entsprechend der orthogonal zueinander angeordneten Scheibenstrukturen 12, 14, 16 durchgeführt werden und lässt sich weitestgehend automatisieren, z.B. mittels einer Bildverarbeitungseinrichtung oder einer Bildverarbeitungssoftware.

Im Folgenden wird die Anwendung des erfindungsgemäßen Verfahrens auf ein in Fig. 3a beispielhaft dargestelltes 3D-Röntgensystem 200 dargestellt. Bezüglich der nachfolgenden Ausführungen wird darauf hingewiesen, dass diese auf i.W. beliebige 3D-Röntgensysteme, wie z.B. CT- oder CL-Systeme etc., anwendbar und übertragbar sind. Fig. 3a zeigt eine Darstellung eines 3D-Röntgensystems 200 mit dem das erfindungsgemäße Verfahren zur Bestimmung der Ortsauflösung mittels des (im vorhergehenden anhand der Fig. 1a beschriebenen) 3D-Prüfkörpers 10 durchgeführt werden kann. Das 3D-Röntgensystem 200 weist beispielsweise eine Röntgenröhre 210 und einen für Röntgenstrahlung 212 empfindlichen (flächigen) Detektor 230, z.B. in Form eines Flachbilddetektors bzw. flächigen Röntgendetektors, auf. Die von der Röntgenstrahlquelle 210 austretende Röntgenstrahlung 212 durchstrahlt bzw. durchdringt den Prüfkörper 10 und trifft auf den röntgenempfindlichen Detektor 230.

Nach Transmission bzw. Durchgang der Röntgenstrahlung 212 durch den Prüfkörper 10 entsteht eine einzelne Mess-Durchstrahlungsaufnahme.

Ferner ist dem 3D-Röntgensystem 200 eine Verarbeitungs- und Steuerungseinrichtung 270 zugeordnet, die zur Ansteuerung des 3D-Röntgensystems 200 einschließlich der Röntgenstrahlungsquelle 210 und des röntgenempfindlichen Detektors 230, zur Ansteuerung der Bewegung des Untersuchungsobjekts, d.h. des Prüfkörpers 10, und ferner zur Auswertung der erfassten Durchstrahlungsbilder dient. In Fig. 3a weist die Röntgendurchstrahlungsaufnahme eine Projektion des dreidimensionalen Volumens des Prüfkörpers 10 auf, wobei die Durchstrahlungsaufnahme bzw. Projektion dadurch entsteht, dass die von der Röntgenstrahlungsquelle 210 austretenden Röntgenstrahlen 212 nach Durchgang durch den Prüfkörper 10 auf die zweidimensionale Oberfläche 232 des röntgenempfindlichen Detektors 230 abgebildet werden. Der röntgenempfindliche Detektor 230 ist beispielsweise als ein Festkörperdetektor ausgebildet und kann als Zeilendetektor, z.B. bei einer Flächenstrahl-CT oder als Mehrzeilen- bzw. Flächendetektor, z.B. bei einer 3D-Konusstrahl-CT, ausgebildet sein. In diesem Zusammenhang wird nochmals darauf hingewiesen, dass das erfindungsgemäße Konzept auf jegliche 3D-Röntgensysteme anwendbar ist, wie z.B. auch auf CL-Systeme etc.

Bei der industriellen CT wird beispielsweise der Projektionsdatensatz, der als Grundlage für die Rekonstruktion von Tiefeninformationen des Prüfkörpers 10 dient, innerhalb einer (oder mehreren) vollen Umdrehung(en) des Prüfkörpers 10 relativ zu dem Röntgensystem in kleinen Winkelschritten akquiriert. Dabei ist das 3D-Röntgensystem aus Röntgenstrahlungsquelle 210 und Röntgendetektor 230 beispielsweise bei einem CT-System relativ zu dem Prüfkörper 10 rotierbar angeordnet. Bei einem CT-System wird beispielsweise abhängig von der Winkelzahl ein 360°-Vollkreis in äquidistanten Winkelpositionen $\delta\alpha$ aufgeteilt, die der Reihe nach angefahren werden. Mittels einer rechnerbasierten Auswertung, die beispielsweise auf der Basis einer mathematischen Transformation (z.B. Radontransformation) durchgeführt werden kann, wird aus der Vielzahl von Mess-Durchstrahlungsaufnahmen (aus dem Durchstrahlungsdatensatz bzw. Projektionsdatensatz) ein 3D-Bild bzw. 3D-Volumen rekonstruiert, wobei jedem Volumenelement bzw. Voxel des 3D-Bildes ein Schwächungskoeffizient bzw. Absorptionsgrad zugeordnet wird.

Die Verarbeitungs- und Steuerungseinrichtung 270 kann beispielsweise ausgebildet sein, um die anhand von Fig. 2 beschriebenen Verfahrensschritte des Erfassens 120 einer Abfolge von Durchstrahlungsaufnahmen des Prüfkörpers an unterschiedlichen Winkelpositionen, des Rekonstruierens 130 einer 3D-Röntgenaufnahme des Prüfkörpers basierend auf der Gesamtzahl von Mess-Durchstrahlungsaufnahmen, und des Bestimmens 140 der Ortsauflösung des 3D-Röntgensystems durchzuführen bzw. zu steuern.

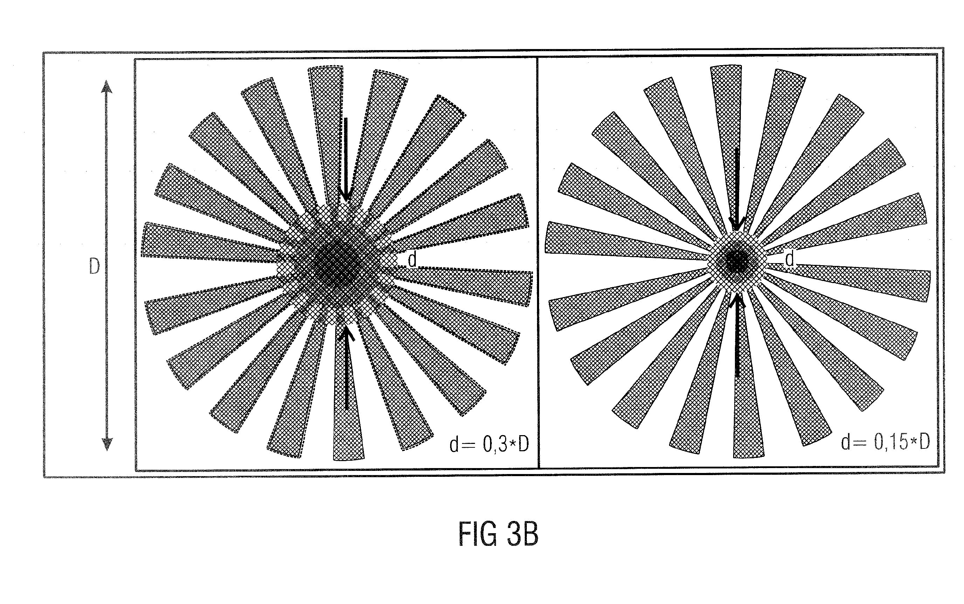

Die Verarbeitungs- und Steuerungseinrichtung 270 des 3D-Röntgensystems kann beispielsweise ferner eine Bildverarbeitungseinrichtung, z.B. eine Bildverarbeitungssoftware, aufweisen, die die Bestimmung der Ortsauflösung des 3D-Röntgensystems basierend auf dem Unschärfe-Bereich der 3D-Röntgenaufnahme automatisch erstellt und ausgibt bzw. anzeigt. Eine solche Ausgabe bzw. Anzeige 270a kann beispielsweise einen ermittelten Wert für die Ortsauflösung "l" und/oder auch eine Darstellung des Unschärfe-Bereichs der 3D-Röntgenaufnahme (vgl. Fig. 3b) aufweisen.

Entsprechend dem erfindungsgemäßen Verfahren 100 zur Bestimmung der Ortsauflösung bzw. des Ortsauflösungsvermögens eines 3D-Röntgensystems wird der erfindungsgemäße Prüfkörper 10 so in dem 3D-Röntgensystem platziert, dass der gesamte 3D-Prüfkörper 10 (EZRT-Stern) im rekonstruierten 3D-Volumen abgebildet wird.

Es ist im Allgemeinen nicht notwendig, dass die Symmetrieebenen des 3D-Prüfkörpers 10, d.h. die orthogonal zueinander angeordneten Scheibenstrukturen 12, 14, 16 des Prüfkörpers 10 parallel zu den Hauptachsen des zu untersuchenden 3D-Röntgensystems liegen. Zur Erhöhung des Kontrasts zwischen den einzelnen Sektoren der Scheibenstrukturen 12, 14, 16 im rekonstruierten 3D-Volumen können die Symmetrieebenen des 3D-Prüfkörpers 10 auch parallel zu den Hauptachsen des zu untersuchenden 3D-Röntgensystems liegen. Die Orientierung bzw. Ausrichtung des erfindungsgemäßen Prüfkörpers 10 kann aber im Wesentlichen beliebig gewählt werden. Darüber hinaus ist eine genaue Ausrichtung mit dem 3D-Röntgensystem bzw. mit der Röntgenquelle und dem Röntgendetektor des 3D-Röntgensystems nicht erforderlich.

Die Bestimmung des Ortsauflösungsvermögens des 3D-Röntgensystems kann für jedes beliebige 3D-bildgebende Verfahren, d.h. für jede Kombination aus Bildaufnahmetrajektorie und Rekonstruktionsmethode, durchgeführt werden.

Aus den 3D-Volumendaten kann nun das Ortsauflösungsvermögen des 3D-Röntgensystems bestimmt werden, indem ein Schnittbild mitten durch eine Scheibenstruktur 12, 14, 16 des Prüfkörpers 10 betrachtet wird. Die Auswertung erfolgt dann durch Bestimmung des Durchmessers des unscharfen Bereichs d in der Mitte des Prüfkörpers 10 Daraus ergibt sich nach folgender Formel das Ortsauflösungsvermögen des 3D-Röntgensystems in Längeneinheiten, wobei N die Anzahl der Sektoren auf einen ganzen 360°-Kreis ist:

$$l = \pi \cdot d/N$$

Die Bestimmung kann mit allen drei aufeinander orthogonal stehenden Scheibenstrukturen durchgeführt werden, wodurch die Ortsauflösung abhängig von der jeweiligen Raumrichtung bestimmt werden kann. Dies ist insbesondere für 3D-Röntgensysteme mit anisotroper Ortsauflösung, d.h. mit unterschiedlichen Ortsauflösungen in unterschiedlichen Richtungen, z.B. bei Computertomographen mit Zeilendetektoren und schichtweiser Abtastung oder laminographischen Aufnahmeverfahren relevant.

Bei der Zeilen-CT mit schichtweiser Abtastung wird die Auflösung innerhalb einer Schicht durch das Röntgensystem (Quelle/Detektor/geometrische Anordnung der Komponenten) bestimmt, während die Auflösung zwischen den einzelnen Schichten vor allem vom Abstand der einzelnen Schichten zueinander abhängt, welcher völlig unabhängig von den restlichen Aufnahmeparametern gewählt werden kann und somit weitestgehend unabhängig von der Auflösung innerhalb einer Schicht ist. Sollen mit einer solchen Röntgenanlage Fehler oder Strukturen mit unterschiedlichen Längenausdehnungen in verschiedene Raumrichtungen erkannt werden, so hängt die Erkennbarkeit des Fehlers bzw. dieser Struktur von deren Orientierung ab, falls die Anlage anisotrope Auflösungseigenschaften aufweist.

Ähnliches gilt für die Computerlaminographie bzw. der Computertomographie mit eingeschränktem Winkelbereich, der sog. (Limited-Angle CT). Bei diesen Verfahren wird das Objekt nur unter einem beschränkten Winkelbereich durchleuchtet. Die beste Ortsauflösung ist in der Regel entlang der Richtung zu erreichen, welche senkrecht auf den meisten Röntgenstrahlen während der Scanvorgangs steht. Die Ortsauflösung in Richtung parallel zu den meisten Röntgenstrahlen ist am schlechtesten. Dies hängt vor allem vom gewählten Winkelbereich ab.

Fig. 3b zeigt nun zwei beispielhafte 3D-Röntgenaufnahmen bzw. Schnittbilder durch jeweils eine Scheibenstruktur des Prüfkörpers mit unterschiedlich resultierenden Ortsauflösungen hinsichtlich einer Raumrichtung eines 3D-Röntgensystems.

Wie in der linken Abbildung von Fig. 3b ersichtlich ist, ergibt sich ein Unschärfe-Bereich, der etwa dem 0,3-fachen Wert des Durchmessers der Scheibenstruktur entspricht. In der rechtseitigen Abbildung von Fig. 3b ergibt sich ein Unschärfe-Bereich, der dem 0,15-fachen Durchmesser der jeweiligen Scheibenstruktur entspricht.

Bezüglich der erfindungsgemäßen Vorgehensweise zur Bestimmung der Ortsauflösung in zumindest einer von drei orthogonalen Raumrichtungen eines 3D-Röntgensystems mittels des Prüfkörpers 10 wird darauf hingewiesen, dass mit dem erfindungsgemäßen Prüfkörper 10 eine Bestimmung der Ortsauflösung des zu untersuchenden 3D-Röntgensystems ohne jegliche Justage oder Ausrichtung des Prüfkörpers 10 hinsichtlich des 3D-Röntgensystems möglich ist. So kann beispielsweise der erfindungsgemäße Prüfkörper 10 ohne weitere Vorbereitung, zusätzliche Infrastruktur oder Justageprozesse auf den Drehteller einer 3D-Röntgenanlage gelegt werden. Alternativ kann auch der erfindungsgemäße 3D-Prüfkörper 10 ganz nach Belieben im Inneren eines Inspektionsobjekts, wie z.B. eines Kraftfahrzeugs oder eines Seecontainers etc. positioniert werden, um das resultierende Ortsauflösungsvermögen des 3D-Röntgensystems zu ermitteln.

Der im Vorhergehenden erläuterte Prüfkörper 10 zur Bestimmung der Ortsauflösung eines 3D-Röntgensystems und das erfindungsgemäße Verfahren 100 zur Bestimmung der Ortsauflösung eines 3D-Röntgensystems unter Verwendung des erfindungsgemäßen Prüfkörpers 10 liefert somit eine Reihe von Vorteilen.

So ist der erfindungsgemäße 3D-Prüfkörper (EZRT-Stern) relativ einfach und somit relativ kostengünstig herzustellen. Die Auswertung des 3D-Prüfkörpers 10 ist einfach und lässt sich weitestgehend automatisieren, z.B. mittels Bildverarbeitungseinrichtungen, wie z.B. einer Bildverarbeitungssoftware. Ferner ist die Positionierung des 3D-Prüfkörpers 10 im Objekt unkritisch, da in den 3D-Volumendaten ggf. Schnittebenen durch die einzelnen Scheibenstrukturen des Prüfkörpers 10 synthetisiert werden können. Der Wert der höchsten Ortsauflösung kann darüber hinaus stufenlos bestimmt werden, wobei dies simultan in drei orthogonalen Raumrichtungen anhand der Aufnahme eines einzigen Volumendatensatzes des Prüfkörpers möglich ist.

Obwohl manche Aspekte im Zusammenhang eines Prüfkörpers zur Bestimmung der Ortsauflösung in zumindest einer von drei orthogonalen Raumrichtungen eines 3D-Röntgensystems beschrieben wurden, versteht es sich, dass diese Aspekte des 3D-Röntgensystems auch eine Beschreibung des entsprechenden Verfahrens zur Bestimmung der Ortsauflösung in zumindest einer von drei orthogonalen Raumrichtungen eines 3D-Röntgensystems darstellen, so dass ein Block oder ein Bauelement einer Vorrichtung, z.B. eines entsprechenden 3D-Röntgensystems, auch als ein entsprechender Verfahrensschritt oder als ein Merkmal eines Verfahrensschritts zu verstehen ist. Analog dazu stellen Aspekte, die im Zusammenhang mit einem oder als ein Verfahrensschritt beschrieben wurden auch eine Beschreibung eines entsprechenden Blocks oder Details oder Merkmals einer entsprechenden Vorrichtung dar. Einige oder alle Verfahrensschritte können auch durch einen Hardware-Apparat (oder unter Verwendung eines Hardware-Apparats), wie z.B. einem Mikroprozessor, einem programmierbaren Computer oder eine elektronische Schaltung ausgeführt werden. Bei einigen Ausführungsbeispielen können einige oder mehrere der wichtigen Verfahrensschritte durch einen solchen Apparat ausgeführt werden.

Je nach bestimmtem Implementierungsaufwand können Ausführungsbeispiele der Erfindung in Hardware oder in Software implementiert sein. Die Implementierung kann unter Verwendung eines digitalen Speichermediums, beispielsweise einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft. Der Programmcode kann beispielsweise auch auf einem maschinenlesbaren Träger gespeichert sein. Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist. Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen. Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**Patentansprüche**

1. Prüfkörper (10) zur Bestimmung der Ortsauflösung eines 3D-Röntgensystems, mit folgenden Merkmalen:

> drei orthogonal zueinander angeordnete und miteinander verbundene Scheibenstrukturen (12, 14, 16) mit einem gemeinsamen Mittelpunkt (18);
> wobei jede Scheibenstruktur eine geradzahlige Anzahl N von Pyramiden- oder Pyramidenstumpf-förmigen Scheibenstruktursektoren (12-N, 14-N, 16-N) aufweist, die sich zu dem gemeinsamen Mittelpunkt (18) hin verjüngen; und
> wobei benachbarte Scheibenstruktursektoren einer Scheibenstruktur unterschiedliche Röntgenstrahlungsabsorptionseigenschaften aufweisen.

2. Prüfkörper nach Anspruch 1, wobei benachbarte Scheibenstruktursektoren einer Scheibenstruktur unterschiedliche Materialien aufweisen.

3. Prüfkörper nach Anspruch 1 oder 2, wobei benachbarte Scheibenstruktursektoren einer Scheibenstruktur abwechselnd einen physischen Scheibenstruktursektor und einen als Materialaussparung ausgebildeten Scheibenstruktursektor aufweisen.

4. Prüfkörper nach einem der vorhergehenden Ansprüche, wobei die Pyramidenoder Pyramidenstumpf-förmigen Scheibenstruktursektoren (12-N, 14-N, 16-N) eine rechteckige oder quadratische Querschnittsfläche aufweisen.

5. Prüfkörper nach einem der vorhergehenden Ansprüche, wobei die Pyramidenoder Pyramidenstumpf-förmigen Scheibenstruktursektoren (12-N, 14-N, 16-N) gleiche geometrische Abmessungen aufweisen.

6. Prüfkörper nach einem der vorhergehenden Ansprüche, wobei jede Scheibenstruktur zumindest 16 Scheibenstruktursektoren aufweist.

7. Prüfkörper nach einem der vorhergehenden Ansprüche, wobei jede Scheibenstruktur zumindest zwei unterschiedliche und bis zu N-unterschiedliche Materialien mit unterschiedlichen Röntgenstrahlungsabsorptionseigenschaften aufweist.

8. Prüfkörper nach einem der vorhergehenden Ansprüche, wobei die Scheibenstruktursektoren (12-N, 14-N, 16-N) Pyramidenstumpf-förmig ausgebildet sind und eine Länge "a" ausgehend von der Grundfläche bis zu der Deckfläche des Pyramidenstumpf-förmigen Scheibenstruktursektors aufweisen, wobei die Länge "a" zumindest 80% des Abstandes zwischen der jeweiligen Grundfläche der Pyramidenstumpf-förmigen Scheibenstruktursektoren bis zu dem gemeinsamen Mittelpunkt entspricht.

9. Prüfkörper nach einem der vorhergehenden Ansprüche, wobei sich die Scheibenstrukturen (12, 14, 16) aufgrund der Pyramiden- oder Pyramidenstumpf-förmigen Scheibenstruktursektoren (12-N, 14-N, 16-N) zum gemeinsamen Mittelpunkt (18) hin verjüngen.

10. Verfahren (100) zur Bestimmung der Ortsauflösung eines 3D-Röntgensystems mittels des Prüfkörpers (10) gemäß einem der vorhergehenden Ansprüche, mit folgenden Schritten:

> Platzieren (110) des Prüfkörpers in einer Prüfposition zwischen einer Röntgenquelle und einem Röntgendetektor des 3D-Röntgensystems;
> Erfassen (120) einer Abfolge von Durchstrahlungsaufnahmen des Prüfkörpers an unterschiedlichen Positionen, um eine Gesamtzahl von MessDurchstrahlungsaufnahmen zu erhalten;
> Rekonstruieren (130) einer 3D-Röntgenaufnahme des Prüfkörpers basierend auf der Gesamtzahl von Mess-Durchstrahlungsaufnahmen; und
> Bestimmen (140) der Ortsauflösung des 3D-Röntgensystems basierend auf dem Durchmesser d eines Unschärfe-Bereichs der 3D-Röntgenaufnahme im Zentrum des Prüfkörpers, wobei innerhalb des Unschärfe-Bereichs keine Auflösung der einzelnen Scheibenstruktursektoren vorliegt, und auf der Anzahl N von Pyramidenoder Pyramidenstumpf-förmigen Scheibenstruktursektoren.

11. Verfahren nach Anspruch 10, wobei die Ortsauflösung "l" des 3D-Röntgensystems basierend auf der folgenden Beziehung ermittelt wird:

$$l = \pi \cdot d/N;$$

mit dem Durchmesser d des Unschärfe-Bereichs der 3D-Röntgenaufnahme im Zentrum des Prüfkörpers und der Anzahl N von Scheibenstruktursektoren pro Scheibenstruktur.

12. Verfahren nach einem der Ansprüche 10 oder 11, wobei der Schritt des Bestimmens (140) der Ortsauflösung in einer Raumebene oder in mehreren orthogonalen Raumebenen der 3D-Röntgenaufnahme entsprechend der orthogonal zueinander angeordneten Scheibenstrukturen durchgeführt wird.

13. Verwenden des Prüfkörpers (10) gemäß einem der Ansprüche 1 bis 9 zur Bestimmung der Ortsauflösung eines 3D-Röntgensystems in zumindest einer von drei orthogonalen Raumrichtungen.

FIG 1A

FIG 1B

A (20:1)

<u>100</u>                                                                                        110

Platzieren des Prüfkörpers in einer Prüfposition zwischen einer Röntgenquelle
und einem Röntgendetektor des 3D-Röntgensystems

                                                                                                120

Erfassen einer Abfolge von Durchstrahlungsaufnahmen des Prüfkörpers
an unterschiedlichen Positionen, um eine Gesamtzahl von
Mess-Durchstrahlungsaufnahmen zu erhalten

                                                                                                130

Rekonstruieren einer 3D-Röntgenaufnahme des Prüfkörpers basierend auf der
Gesamtheit von Mess-Durchstrahlungsaufnahmen

                                                                                                140

Bestimmen der Ortsauflösung des 3D-Röntgensystems basierend auf dem
Durchmesser d eines Unschärfe-Bereichs der 3D-Röntgenaufnahme im Zentrum des Prüfkörpers, wobei innerhalb des Unschärfe-Bereichs keine Auflösung
der einzelnen Scheibenstruktursektoren vorliegt, und auf der Anzahl N von
Pyramiden- oder Pyramidenstumpf-förmigen Scheibenstruktursektoren

## FIG 2

Fig 3A

FIG 3B

**EUROPÄISCHER RECHERCHENBERICHT**

Europäisches Patentamt
European Patent Office
Office européen des brevets

Nummer der Anmeldung

EP 14 17 8229

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 10 2006 032607 A1 (ZEISS IND MESSTECHNIK GMBH [DE]) 17. Januar 2008 (2008-01-17) * Absatz [0036] - Absatz [0041]; Abbildungen 6-8 * ----- | 1-13 | INV. A61B6/00 G01N23/04 |
| A | SASOV, ALEXANDER ET. AL.: "New type of x-ray source for lensless laboratory nano-CTwith 50nm resolution", PROC. SPIE 7804, Bd. 7804, 78040Q, 2. September 2010 (2010-09-02), Seiten 1-8, XP040543563, San Diego, California DOI: 10.1117/12.860340 * das ganze Dokument * ----- | 1-13 | |
| A | DE 10 2011 001746 A1 (WERTH MESSTECHNIK GMBH [DE]) 4. Oktober 2012 (2012-10-04) * das ganze Dokument * ----- | 1-13 | |
| A | US 2003/223550 A1 (KIM JAE-WAN [KR] ET AL) 4. Dezember 2003 (2003-12-04) * das ganze Dokument * ----- | 1-13 | RECHERCHIERTE SACHGEBIETE (IPC) G01N A61B |
| A | US 4 055 771 A (GOODENOUGH DAVID JOHN ET AL) 25. Oktober 1977 (1977-10-25) * Spalte 5, Zeile 66 - Spalte 6, Zeile 25; Abbildung 13 * ----- | 1-13 | |

Der vorliegende Recherchenbericht wurde für alle Patentansprüche erstellt

| Recherchenort | Abschlußdatum der Recherche | Prüfer |
|---|---|---|
| Den Haag | 23. Oktober 2014 | Pagels, Marcel |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 14 17 8229

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-10-2014

| Im Recherchenbericht angeführtes Patentdokument | Datum der Veröffentlichung | Mitglied(er) der Patentfamilie | Datum der Veröffentlichung |
|---|---|---|---|
| DE 102006032607 A1 | 17-01-2008 | DE 102006032607 A1<br>EP 2038642 A2<br>JP 5417172 B2<br>JP 2009543083 A<br>US 2009242744 A1<br>WO 2008006569 A2 | 17-01-2008<br>25-03-2009<br>12-02-2014<br>03-12-2009<br>01-10-2009<br>17-01-2008 |
| DE 102011001746 A1 | 04-10-2012 | KEINE | |
| US 2003223550 A1 | 04-12-2003 | JP 3717491 B2<br>JP 2004012459 A<br>KR 20030093528 A<br>US 2003223550 A1 | 16-11-2005<br>15-01-2004<br>11-12-2003<br>04-12-2003 |
| US 4055771 A | 25-10-1977 | JP S5353993 A<br>US 4055771 A | 16-05-1978<br>25-10-1977 |

EPO FORM P0461

Für nähere Einzelheiten zu diesem Anhang : siehe Amtsblatt des Europäischen Patentamts, Nr.12/82